# EUROPEAN PATENT APPLICATION

(11) **EP 3 502 684 A1**
(43) Date of publication of application: **26.06.2019**
(21) Application number: 17306908.9
(22) Date of filing: 22.12.2017
(51) Int. Cl.: G01N 31/22, G01N 33/18

(54) **METHOD FOR METAL ION DETECTION IN AQUEOUS SOLUTIONS USING NUCLEOLIPID COMPOUNDS**

(71) Applicant: UNIVERSITE DE BORDEAUX, 33000 Bordeaux (FR); CENTRE NATIONAL DE LA RECHERCHE SCIENTIFIQUE (CNRS), 75016 Paris (FR); INSTITUT NATIONAL DE LA SANTE ET DE LA RECHERCHE MEDICALE (INSERM), 75013 Paris (FR)
(72) Inventor: BARTHELEMY, Philippe, 33700 MERIGNAC (FR); ALIES, Bruno, 33000 BORDEAUX (FR); OUELHAZI, Mohamed Amine, 33700 MERIGNAC (FR)
(74) Representative: Santarelli

(57) **Abstract**

The invention relates to a method for metal ion detection in aqueous solutions, in particular silver or mercury, using nucleolipid compounds, and to new nucleolipid compounds which can be used in said method.

## Description

The invention relates to a method for metal ion detection in aqueous solutions using nucleolipid compounds, and to new nucleolipid compounds which can be used in said method.

Detecting metals under different forms is a global public health issue. Numerous metals are under surveillance, such as, for instance, aluminum, antimony, arsenic, cadmium, chrome, copper, tin, iron, manganese, mercury, nickel, lead, selenium, thallium, vanadium and zinc, with respect to their carcinogenic or non-carcinogenic acute or chronic effects.

These effects are registered at the international level, for instance, at the US Agency ATSDR (Agency for Toxic Substances and Disease Registry) or at the US EPA (United States Environmental Protection Agency) - IRIS (Integrated Risk Information System) database , or else at the IARC - CIRC (International Agency for Research on Cancer, World Health Organization).

Silver is well-known for its antibacterial properties since ancient time. Nowadays, silver nanoparticles are the major form of silver, as they have become widely used from hospital to kitchen. Nanoparticles end up to be a concerning contaminant, even if they are usually considered to be weakly toxic. Nevertheless, their oxidation can produce harmful metal ions for environment and human beings.

Silver ions (Ag⁺) are not toxic at low concentration and exhibit interesting properties, such as a microbicidal activity, for example in drinking water, at a concentration lower than 100µg/L, but may become toxic to aquatic organisms and humans at high concentrations.

There is thus a need for a selective and reliable method for detecting metal ions in the environment, in particular in aqueous solutions.

Nucleotide amphiphiles known as nucleolipids, which are biocompatible structures composed of a lipid covalently attached to a nucleotide, display a low toxicity and have demonstrated their usefulness from biocompatible gel to medical imaging, or in nanoparticle formulations useful for drug delivery.

For example, WO2009/098404 and WO2010/136676 relate to the preparation and uses of such compounds for transportation or vectorization of therapeutic agents. WO 2016/170010 relates to non-polymeric lipid-based nanocarrier compositions loaded with metal nanoparticles and a therapeutic agent, as agents for transportation, vectorization, cellular delivery cellular targeting or cellular localization of said therapeutic agent.

It has now been found that nucleolipids can be used to detect metal ions in aqueous solutions, in particular silver or mercury ions.

It was also found that, depending especially on the purine or pyrimidine base present in the nucleolipid structure, the detection method could be monitored with respect to ion selectivity and/or to sensitivity of the measurement.

A method for silver (I) ion detection in water, drugs and food, involving an oligonucleotide (OND) probe containing cytosine moieties labeled at the 5' end with a fluorescent label, and linked to a guanine (G) quadruplex having inherent quenching ability at the 3'end is disclosed in L. Bian et al., J. Agric Food Chem, 2014, 62, 4870-4877. Upon interaction with silver (I) ions, the OND folds into a hairpin structure, whereby the fluorescence of the fluorescent label is quenched by the G-quadruplex.

K. Jang et al., New J Chem, 2017, 41, 1840 relates to the detection of silver ions using a silver-specific poly(cytosine)DNA immobilized on a nanoporous micro-resonator (NPMR) and single cytosine. Silver ions are detected by measuring the resonance frequency shift of the NPMR by the interaction of silver ions with cytosine bases of silver-specific poly(cytosine)DNA and cytosine molecules.

The detection of silver ions (Ag⁺) by the fluorescence switch of DNA-templated silver nanoclusters triggered by Ag⁺ in air saturated di-ionized water is disclosed in J. Lee et al., Biosensors and bioelectronics, 2015, 68, 642.

These methods rely on complex systems where Ag+ sensing is based on oligonucleotide or DNA-like probes. However, these systems are very costly and are not fully convenient because of, for instance, the fragility and instability of these molecules. Also, these systems do not form supra-molecular assemblies (gels).

It has now been found that nucleolipids, which are able to self-assemble into stable liposomes, can be used to detect metal ions in aqueous solutions.

Without wishing to be bound by theory, it can be hypothesized that nucleolipids spontaneously form stable liposomes or self-assemblies which display a pyridine or pyrimidine base layer at the surface of liposome. This pyridine or pyrimidine base layer could interact with or bind the metal ion, and the coordination of the metal ion increases the rigidity of the membrane. By using a common luminescent probe, for example a fluorescent probe, the variation of the supramolecular structure induced by the metal ion makes it possible to detect the presence of the metal ion, even at low concentration, with great specificity.

Said metal ion can be, for example, aluminum, antimony, arsenic, cadmium, chrome, cobalt, copper, tin, iron, manganese, mercury, nickel, lead, selenium, silver, thallium, vanadium, zinc, etc.

In the present description, the terms "liposome" or "self-assembly" or "self-assembled structure" will be equally used.

The invention thus relates to a method for metal ion detection in aqueous solutions, comprising :
- contacting an aqueous solution containing at least one metal ion with at least one nucleolipid compound of formula (I) 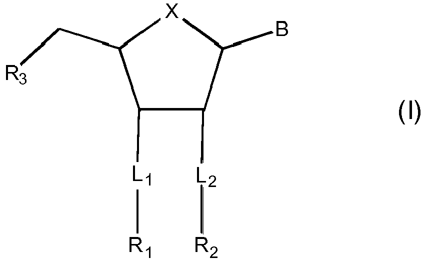 in which
- X is an oxygen atom, a sulfur atom or a methylene group,
- B is a purine or pyrimidine base, or else a non-natural mono- or bi-cyclic heterocyclic base, each ring of which comprises 4 to 7 members, optionally substituted ;
- L₁ and L₂, identical or different, represent hydrogen, an oxycarbonyl -O-C(O)- group, a thiocarbamate -O-C(S)-NH- group, a carbonate -O-C(O)-O-group, a carbamate -O-C(O)-NH- group, an oxygen atom, a phosphate group, a phosphonate group or a heteroaryl group comprising 1 to 4 nitrogen atoms, unsubstituted or substituted by a linear or branched, saturated or unsaturated C₈-C₃₀ hydrocarbon chain, wherein L₁ and L₂ are not simultaneously hydrogen,
- or L₁ represents a phosphate or phosphonate group and L₂ represents hydrogen;
- or also, L₁ and L₂, together, form a ketal group of formula 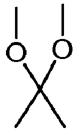
- or also L₁ or L₂ represents hydrogen, and the other represents a hydroxy group or a heteroaryl group comprising 1 to 4 nitrogen atoms, unsubstituted or substituted by a linear or branched C₈-C₃₀, alkyl chain,
- R₁ and R₂, identical or different, represent
- a linear or branched C₈-C₃₀ hydrocarbon chain, saturated or partially unsaturated, or
- a C₈-C₃₀ acyl chain,
- a diacyl chain in which each acyl chain is C₈-C₃₀,
- a diacylglycerol in which each acyl chain is C₈-C₃₀, or
- a sphingosine or ceramide group in which each acyl chain is C₈-C₃₀, or
- when L₁ or L₂ represents hydrogen, and the other represents a hydroxy group or a heteroaryl group comprising 1 to 4 nitrogen atoms, R₁ and R₂ do not exist;
- R₃ represents
- a hydroxy, amino, phosphate, phosphonate, phosphatidylcholine, O-alkyl phosphatidylcholine, thiophosphate, phosphonium, NH₂-R₄, NHR₄R₅ or NR₄R₅R₆ group in which R₄, R₅ and R₆, identical or different, represent a hydrogen atom or a linear or branched C₁-C₆ alkyl chain or linear or branched C₁-C₆ hydroxyalkyl, or
- a linear or branched C₂-C₃₀ alkyl chain, optionally substituted by a hydroxy group, or
- a heteroaryl group containing 1 to 4 nitrogen atoms, unsubstituted or substituted by a C₂-C₃₀ alkyl, optionally substituted by a hydroxy group; or by a (CH₂)ₘ-O-(CH₂)ₚ-R₉ group in which m = 1 to 6 and p = 0 to 20 and R₉ represents hydrogen or a cyclic ketal group containing 5 to 7 carbon atoms, unsubstituted or substituted by at least one linear or branched C₂-C₃₀ alkyl, or by a sterol radical,
   or
- a -O-C(O)-(CH₂)_{q}-C(O)-O [(CH₂)₂-O]ᵣ-H group in which q is an integer from 2 to 6 and r is an integer from 4 to 30, preferably from 10 to 20, or also
- R₃ is bound by a covalent bond to another substituent R₃, identical or different, of another compound of formula (I), identical or different, in order to form a compound of formula (I) in the form of a dimer,
   and
- detecting the presence of the at least one metal ion by measuring the interaction of said at least one metal ion with said at least one nucleolipid compound of formula (I).

The invention also relates to the use of compounds of formula (I) as defined above for metal ion detection in aqueous solutions.

In particular, said interaction consists in the binding of said at least one metal ion to said at least one nucleolipid compound of formula (I).

In particular, the nucleolipid compound of formula (I), which possesses self-assembly properties, forms a liposome structure and the metal ion(s) interacts with the purine or pyrimidine base moiety of the compounds of formula (I).

Preferably, the nucleolipid compound of formula (I), which possesses self-assembly properties, forms a liposome structure and the metal ion(s) binds to the purine or pyrimidine base moiety of the compounds of formula (I).

By "an aqueous solution containing at least one metal ion" is understood any aqueous medium or sample thereof where the presence of at least one metal ion is looked for.

For example, said aqueous medium or sample thereof can be water intended for human consumption, in particular drinking water; water used in the food or pharmaceutical industry; water bodies (such as ponds or lakes); coastal waters; surface water, groundwater and river water; river water for irrigation; lakewater or seawater for aquaculture; industrial waters, in particular industrial waste waters; mining waters; bathing waters, in particular water for swimming pools etc.; where the amount of metal ion(s) needs to be controlled or assessed.

Advantageously, said metal ion can be detected at a concentration of 0.05 µM/L to 5 µM/L (5 ppb to 500 ppb).

Said metal ion can be detected by the method of the invention, advantageously, in a molar ratio of nucleolipid/metal ion of, for example, 0.5 to 10/1, preferably of 4/1.

The presence of the at least one metal ion can be detected, for example, by using an indicator compound, such as a luminescent probe, in particular a fluorescent probe.

Fluorescent probes can be, for example, selected from Thioflavin T, Congo red, Crysamin G, etc., which are usual in the field.

In particular, the presence of the at least one metal ion which interacts with, in particular binds to, a nucleolipid compound of formula (I) results in an alteration of the emitted luminescence, for example an increase of the emitted luminescence, in comparison to that of an aqueous solution containing a nucleolipid compound of formula (I) as defined above and a luminescent probe.

Alternatively, the presence of the at least one metal ion which interacts with, in particular binds to, a nucleolipid compound of formula (I) results in a decrease of the emitted luminescence, in comparison to that of an aqueous solution containing an nucleolipid compound of formula (I) as defined above and a luminescent probe.

In a preferred embodiment, the invention relates to a method for metal ion detection in aqueous solutions, comprising :
- contacting an aqueous solution containing at least one metal ion with a nucleolipid compound of formula (I) as defined above and a luminescent probe, and
- measuring the emitted luminescence,
wherein said emitted luminescence results from the interaction of said at least one metal ion with said at least one nucleolipid compound of formula (I).

In particular, said emitted luminescence results from the binding of said at least one metal ion to said at least one nucleolipid compound of formula (I).

Preferably, the method for metal ion detection in aqueous solutions according to the invention comprises :
- contacting a sample of an aqueous solution containing at least one metal ion with at least one nucleolipid compound of formula (I) as defined above and a luminescent probe,
- measuring the emitted luminescence,
- comparing the emitted luminescence with that of a control sample of an aqueous solution containing at least one nucleolipid compound of formula (I) and a luminescent probe,
wherein the change of emitted luminescence results from the interaction of said at least one metal ion with said at least one nucleolipid compound of formula (I).

In particular, said change of emitted luminescence results from the binding of said at least one metal ion to said at least one nucleolipid compound of formula (I).

By "change of emitted luminescence" is understood an alteration of the luminescence in comparison to that of an aqueous solution containing an nucleolipid compound of formula (I) as defined above and a luminescent probe.

Preferably, in the above methods for metal ion detection, the luminescent probe is a fluorescent probe.

The purine or pyrimidine base of the compound of formula (I) can be, for example, selected from, adenine, guanine, cytosine, xanthine, hypoxanthine, uric acid, caffeine, theobromine, uracile, thymine, dihydrouridine, and their derivatives.

Cytosine, thymine, guanosine and adenine are preferred.

Also, in formula (I) above, the purine or pyrimidine base can be substituted by at least one substituent selected from, for example, a halogen, an amino group, a carboxy group, a carbonyl group, a carbonylamino group, a hydroxy, azido, cyano, thiol, a C₁-C₆ straight or branched alkyl, cycloalkyl, perfluoroalkyl, alkyloxy (for example, methoxy), oxycarbonyl, vinyl, ethynyl, propynyl, acyl group etc.

By "derivatives of a purine or pyrimidine base" is meant, for example, a non-natural mono- or bi-cyclic heterocyclic base in which each cycle has 4 to 7 members, optionally substituted as stated above for the purine or pyrimidine base.

By « non-natural heterocyclic base » is meant a universal base, such as, for example, 3-nitropyrrole, 4-nitroimidazole or 5-nitroindole, which does not exist in nature.

By « heteroaryl comprising 1 to 4 nitrogen atoms » is meant a mono-or bi-cyclic carbocyclic group, aromatic or partially unsaturated, comprising 5 to 12 atoms in total, interrupted by 1 to 4 nitrogen atoms, which can be, for example, selected from furane, pyrrole, oxazole, oxadiazole, isoxazole, pyrazole, triazole, tetrazole, imidazole, pyridine, pyrimidine, pyridazine, pyrazine, benzofurane, indole, quinoleine, isoquinoleine, chromane, naphtyridine and benzodiazine groups, triazole being preferred.

The acyl chain, whenever it occurs, is a C₈-C₃₀, more preferably C₈-C₂₆, even more preferably C₁₆-C₂₀ acyl chain.

Where appropriate, the counter-ion may be chosen, for example, among monovalent cations, such as Na⁺, Li⁺, K⁺, NH₄⁺, Na⁺ being preferred.

According to the invention, the following nucleolipid compounds of formula (I), in which at least one condition is fulfilled, are preferred:
- X is an oxygen atom;
- B is cytosine, thymine, guanosine or adenine;
- L₁ is a phosphate group which is substituted by a R₁ group where where R₁ is diacylglycerol in which each acyl group is C₈-C₃₀, more preferably C₈-C₂₆, even more preferably C₁₆-C₂₀,
- L₂ is hydrogen, and
- R₃ is hydroxy.

Particularly preferred compounds of formula (I) are selected from
- Thymidine 3'-(1,2-dipalmitoyl-*sn*-glycero-3-phosphate), also called diC16-3'-dT (CAS Registry Number: 1160002-70-9);
- (2*R*, 3*S*, 5*R*)-5-(2-amino-6-oxo-1,6-dihydro-9*H*-purin-9-yl)-2-(hydroxymethyl)tetrahydrofuran-3-yl ((*R*)-2,3-bis(palmitoyloxy)propyl)phosphate, also called diC16-3'-dG; and
- (2*R*, 3*S*, 5*R*)-5-(4-amino-2-oxopyrimidin-1(2*H*)-yl)-2-(hydroxymethyl)tetrahydrofuran-3-yl ((*R*)-2,3-bis(tetradecanoyloxy)propyl)phosphate, also called diC16-3'-dC.

The compounds of formula (I) in which
- X is an oxygen atom;
- B is cytosine or guanosine;
- L₁ is a phosphate group which is substituted by a R₁ group where where R₁ is diacylglycerol in which each acyl group is C₁₆,
- L₂ is hydrogen, and
- R₃ is hydroxy,
are new compounds which are an object of the invention.

Namely, (2*R*, 3*S*, 5*R*)-5-(2-amino-6-oxo-1,6-dihydro-9H-purin-9-yl)-2-(hydroxymethyl)tetrahydrofuran-3-yl ((*R*)-2,3-bis(palmitoyloxy)propyl)phosphate (diC16-3'-dG), and
(2*R*, 3*S*, 5*R*)-5-(4-amino-2-oxopyrimidin-1(2*H*)-yl)-2-(hydroxymethyl)tetrahydrofuran-3-yl ((*R*)-2,3-bis(tetradecanoyloxy)propyl)-phosphate (diC16-3'-dC)
are new compounds which are a further object of the invention.

In particular, the invention relates to a method for metal ion detection in aqueous solutions, in which:
- the metal ion is silver (Ag+) and the compound of formula (I) is (2*R*, 3*S*, 5*R*)-5-(4-amino-2-oxopyrimidin-1(2*H*)-yl)-2-(hydroxymethyl)tetrahydrofuran-3-yl ((*R*)-2,3-bis(tetradecanoyloxy)propyl)phosphate, also called diC16-3'-dC ; or
- the metal ion is mercury (Hg²⁺) and the compound of formula (I) is Thymidine 3'-(1,2-dipalmitoyl-*sn*-glycero-3-phosphate), also called diC16-3'-dT, or
- the metal ion is mercury (Hg²⁺) or copper (Cu²⁺) and the compound of formula (I) is (2*R*, 3*S*, 5*R*)-5-(2-amino-6-oxo-1,6-dihydro-9H-purin-9-yl)-2-(hydroxymethyl)-tetrahydrofuran-3-yl ((*R*)-2,3-bis(palmitoyloxy)propyl)-phosphate , also called diC16-3'-dG.

For the preparation of the compounds of formula (I), reference can be made to WO 2005/116043, which describes different access routes to this type of compounds (see in particular pp. 8-17 and the examples), as well as to WO2009/098404 or WO 2010/136676.

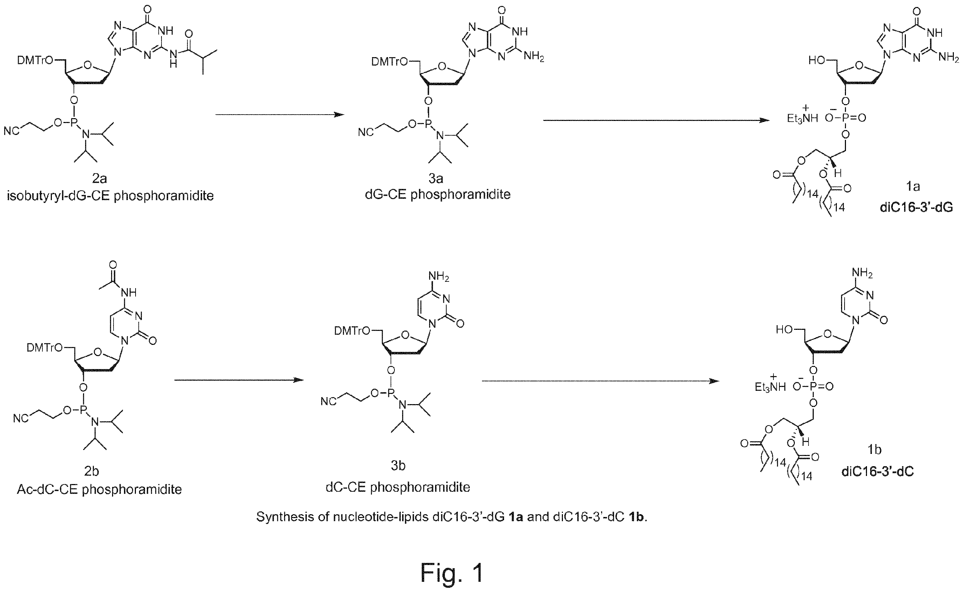

The new compounds of formula (I) diC16-3'-dG and diC16-3'-dC were synthesized as shown on the synthesis scheme of Figure 1 (compounds **1a** and **1b)**.

Briefly, commercially available isobutyryl protected guanosine phosphoramidite **2a** (isobutyryl-dG-CE phosphoramidite)) and acetyl protected cytosine phosphoramidite **2b** (Ac-dC-CE phosphoramidite) were deprotected with methylamine solution in THF to achieve dG-CE phosphoramidite **3a** and dC-CE phosphoramidite **3b**, respectively.

These phosphoramidites (**3a** and **3b**) were coupled with 1,2-dipalmitoyl-sn-glycerol in the presence of a tetrazole solution in acetonitrile (weakly acidic condition) to provide intermediate coupling product. The resulting phosphite intermediates were oxidized, with iodine solution in THF/pyridine/water mixture, to obtain cyanoethyl protected phosphate intermediate. After deprotection of the dimethoxytrityl (5'-DMTr) group under acidic conditions (trichloroacetic acid in dichloromethane DCM), the removal of the cyanoethyl chain was achieved under basic conditions (triethylamine) to yield diC16-3'-dG **1a** and diC16-3'-dC **1b** as their triethylammonium salts.

The nucleolipids featuring thymidine (diC16-3'-dT **1c**) and adenosine (diC16-3'-dA **1d**) can be synthesized according to the literature procedure (S. Khiati et al., Bioconjugate Chem., 2009, 20, 1765-1772 for diC16-3'-dT and B. Desbat et al., Langmuir, 2012, 28, 6816-6825 for diC16-3'-dA).

The invention is illustrated by the examples below.
Example 1 relates to the preparation of diC16-3'-dG (new compound of formula (I)).
Example 2 relates to the preparation of diC16-3'-dC (new compound of formula (I)).
Exemple 3 relates to the preparation of diC16-3'-dC liposomes.
Example 4 relates to the method of detecting metal ions in an aqueous solution.
   Figure 1 represents the synthesis scheme of diC16-3'-dG and diC16-3'-dC.
   Figure 2 shows the DLS profiles of diC16-3'-dC and diC16-3'-dG (intensity-averaged diameter).
   Figure 3 represents the measure of ThT fluorescence for a concentration range of Ag+ in presence of diC16-3'-dC liposomes.
   Figure 4 represents the measure of the THT fluorescence emission with diC16-3'-dC liposomes in the presence of different metal ions.
   Figure 5 represents the measure of the THT fluorescence emission with different diC16-3'-dC or diC16-3'-dT liposomes in the presence of Ag⁺ or Hg²⁺.

All compounds were purchased from *Sigma-Aldrich, Fluka* and *Alfa Aesar* unless otherwise mentioned. Solvents for reactions were purchased from *Sigma-Aldrich* in the highest quality and from *VWR* for other uses. All the reactions were run under nitrogen atmosphere unless otherwise stated. Analytical thin layer chromatography (TLC) was performed on pre-coated silica gel F₂₅₄ plates with fluorescent indicator from *Merck.*

The detection of compounds was accomplished using a UV light (254 nm) and visualized on TLC plates by subsequent spraying with 10 % conc. H₂SO₄ solution in ethanol, followed by heating.

Column chromatography was performed with flash silica gel (0.04-0.063 mm) from *Merck.*

All the compounds were characterized using ¹H, ¹³C and ³¹P Nuclear Magnetic Resonance (NMR) spectroscopy. These NMR spectra were recorded (either in CDCl₃ obtained from *Eurisotop*) on BRUKER Avance DPX-300 spectrometer (¹H at 300.13 MHz, ¹³C at 75.46 MHz and ³¹P at 121.49 MHz). The chemical shifts (*δ*) are given in parts per million (ppm) relatively to tetramethylsilane or residual solvent peaks (CHCl₃: ¹H: 7.26, ¹³C: 77.0). The coupling constants *J* are given in Hertz (Hz); the peak multiplicity is reported as follows: s = singlet, bs = broad singlet, d = doublet, t = triplet, m = multiplet.

### Example 1 : Synthesis of triethylammonium (2R,3S,5R)-5-(2-amino-6-oxo-1,6-dihydro-9H-purin-9-yl)-2-(hydroxymethyl)tetrahydrofuran-3-yl ((R)-2,3-bis(palmitoyloxy)propyl) phosphate (diC16-3'-dG) (compound 1a)

The synthesis scheme is shown on Figure 1.
a) *Step 1 (deprotection of isobutyryl group):* (2*R*,3*S*,5*R*)-2-((bis(4-methoxyphenyl)(phenyl)methoxy)methyl)-5-(2-isobutyramido-6-oxo-1,6-dihydro-9*H*-purin-9-yl)tetrahydrofuran-3-yl (2-cyanoethyl) diisopropyl-phosphoramidite (isobutyryl-dG-CE phosphoramidite) **2a** (2 g, 2.38 mmol, 1 equiv.) was dissolved in MeNH₂ (2 M in THF, 23.81 mL, 47.6 mmol, 20 equiv.) at room temperature under argon. After being stirred for 14 h, the mixture was evaporated under reduced pressure. The completion of the reaction was confirmed by TLC (disappearance of the spot corresponds to starting material and appearance of a new spot at slightly lower retardation factor (*R_{f}*)) The residue was chromatographed on a column of silica gel with 1% MeOH/DCM (v/v) containing 1% TEA and then 3% MeOH/DCM (v/v) containing 1% TEA to give the fractions containing the target (2*R*,3*S*,5*R*)-5-(2-amino-6-oxo-1,6-dihydro-9*H*-purin-9-yl)-2-((bis(4-methoxyphenyl)(phenyl)methoxy)methyl)-tetrahydrofuran-3-yl (2-cyanoethyl) diisopropylphosphoramidite **3a**.
   Yield: 1.33 g (72.7 %). The product was utilized for next step without further characterization.
b) *Step 2 (coupling with lipid):* (2*R*,3*S*,5*R*)-5-(2-amino-6-oxo-1,6-dihydro-9*H*-purin-9-yl)-2-((bis(4-methoxyphenyl)(phenyl)methoxy)methyl)-tetrahydrofuran-3-yl (2-cyanoethyl) diisopropylphosphoramidite **3a** (product of step 1) (1.2 g, 1.64 mmol, 1 equiv.) and 1,2-dipalmitoyl-*sn*-glycerol (1.21 g, 2.14 mmol, 1.3 equiv,) were dissolved in dry THF (25 mL). A tetrazole solution in acetonitrile (0.45 M, 6.2 mL, 2.8 mmol, 1.7 equiv.) was added, and the reaction mixture was stirred overnight at room temperature followed by oxidation with I₂ solution (0.02 M in THF/Pyridine/H₂O, 140 mL, 2.8 mmol, 1.7 equiv.). After 6 h at room temperature, the solvent was evaporated under high vacuum and the intermediate products were dissolved in ethyl acetate (100 mL) and then washed with saturated Na₂S₂O₃ solution (2 x 25 mL). The fractions were dried over anhydrous sodium sulfate, and the solvent was evaporated. The resulting product was dissolved in DCM (20 mL) and trichloroacetic acid (TCA, 3% in DCM) solution (15 mL) under argon, after 4 h at room temperature, 5 mL of methanol and 25 mL of sodium hydrogen carbonate solution were added. The aqueous phase was extracted with DCM (2 x 30 mL), and then the organic fractions were collected, dried over anhydrous sodium sulfate, and the solvent evaporated. The intermediate product was dissolved 33% trimethylamine (TEA) in DCM (50 mL DCM + 25 mL TEA) and stirred overnight at room temperature (to ensure the complete deprotection of cyanoethyl chain). The solvent was evaporated under high vacuum.

The product **1a** was isolated after purification on silica gel (DCM:methanoI:TEA from 98:1:1 to 90:9:1). Yield: 852 mg (32.8 %). *R_{f}* (product **1a**) = 0.3 (DCM:methanoI:TEA 90:9:1).

**¹H NMR** (300 MHz, CDCl₃): *δ* in ppm 0.86 (t, 6H, *J*=6.6 Hz, 2CH₃ of palmitoyl chain), 1.23 (s, 48H, 24CH₂ (palmitoyl chain)), 1.33 (t, 9H, *J*=7.2 Hz, 3CH₃ (triethylammonium)), 1.57 (bs, 4H, 2*CH*₂-CH₂-CO), 2.13-2.40 (m, 4H, 2*CH₂*-CO), 2.54 (bs, 1 H, H2'(sugar)), 2.87 (bs, 1 H, H2"(sugar)), 3.11 (q, *J*=7.1, 3CH₂ (triethylammonium)), 3.64-4.49 (m, 8H, 2CH₂ (glycerol), H3' (sugar), H4'(sugar), 2H5'(sugar)), 5.03-5.36 (m, 2H, -CH- glycerol, -OH), 6.16 (t, 1 H, *J*=6.8 Hz, H1'), 6.52 (bs, 2H, -NH₂), 7.67 (s, 1H, H-8(base)), 12.42 (s, 1H, - NH(base)).

**¹³C NMR** (75 MHz, CDCl₃): *δ* in ppm 8.7 (CH₃, triethylammonium), 14.2 (CH₃ chain), 22.8 (*CH₂*-CH₃ chain), 25.0 (*CH₂*-CH₂-C=O), 29.2-29.8 (CH₂ chain), 32.0 (*CH₂*-CH₂-CH₃ chain), 34.2 (*CH₂*-C=O chain), 34.4 (*CH₂*-C=O chain), 45.9 (CH₂ triethylammonium), 62.7(*CH₂*-O-C=O glycerol), 63.2 (*CH₂-*O-P=O glycerol), 63.7 (CH₂, C5'-sugar), 70.35 (CH, C3'-sugar), 70.44 (CH glycerol), 86.7 (C, C4'-sugar), 87.7 (CH, C1'-sugar), 118.6 (C, C5-base), 137.0 (CH, C8-base), 149.9 (C, C4-base), 154.0 (C, C2-base), 158.2 (C=O, C6-base), 173.2 (C=O chain), 173.5 (C=O chain).

**³¹P NMR** (121 MHz, CDCl₃): *δ* in ppm 1.86. High-resolution ESI MS [M-H]⁻, theoretical m/z=896.55192, observed m/z=896.5525 (1 ppm).

### Example 2 : Synthesis of triethylammonium (2R,3S,5R)-5-(4-amino-2-oxopyrimidin-1(2H)-yl)-2-(hydroxymethyl)tetrahydrofuran-3-yl ((R)-2,3-bis(tetradecanoyloxy)propyl) phosphate (diC16-3'-dC) (compound 1b)

The synthesis scheme is shown on Figure 1.
a) *Step 1 (deprotection of isobutyryl group):*
   (2R,3S,5R)-5-(4-acetamido-2-oxopyrimidin-1 (2H)-yl)-2-((bis(4-methoxyphenyl)(phenyl)-methoxy)methyl)tetrahydrofuran-3-yl (2-cyanoethyl) diisopropyl-phosphoramidite (Ac-dC-CE phosphoramidite) **2b** (2 g, 2.6 mmol, 1 equiv.) was dissolved in MeNH₂ (2 M in THF, 25.9 mL, 51.8 mmol, 20 equiv.) at room temperature under argon. After being stirred for 2 h, the mixture was evaporated under reduced pressure. The completion of the reaction was confirmed by TLC (disappearance of the spot corresponds to starting material and appearance of a new spot at slightly lower retardation factor (*R_{f}*)). The residue was chromatographed on a column of silica gel with 1% MeOH/DCM (v/v) containing 1% TEA and then 3% MeOH/DCM (v/v) containing 1% TEA to give the fractions containing the target (2*R*,3*S*,5*R*)-5-(4-amino-2-oxopyrimidin-1(2*H*)-yl)-2-((bis(4-methoxyphenyl)(phenyl)methoxy)methyl)tetrahydrofuran-3-yl (2-cyanoethyl) diisopropylphosphoramidite **3b.**
   Yield: 1.68 g (89.2 %). The product was utilized for next step without further characterization.
b) *Step 2 (coupling with lipid):* (2*R*,3*S*,5*R*)-5-(4-amino-2-oxopyrimidin-1(2*H*)-yl)-2-((bis(4-methoxyphenyl)(phenyl)methoxy)methyl)-tetrahydrofuran-3-yl (2-cyanoethyl) diisopropylphosphoramidite **3b** (product of step 1) (1.4 g, 1.81 mmol, 1 equiv.) and 1,2-dipalmitoyl-sn-glycerol (1.34 g, 2.36 mmol, 1.3 equiv,) were dissolved in dry THF (25 mL). A tetrazole solution in acetonitrile (0.45 M, 6.9 mL, 3.1 mmol, 1.7 equiv.) was added, and the reaction mixture was stirred overnight at room temperature followed by oxidation with I₂ solution (0.02 M in THF/Pyridine/H₂O, 154 mL, 3.1 mmol, 1.7 equiv.). After 6 h at room temperature, the solvent was evaporated under high vacuum and the intermediate products were dissolved in ethyl acetate (100 mL) and then washed with saturated Na₂S₂O₃ solution (2 x 25 mL). The fractions were dried over anhydrous sodium sulfate, and the solvent was evaporated. The resulting product was dissolved in DCM (20 mL) and trichloroacetic acid (TCA, 3% in DCM) solution (15 mL) under argon, after 4 h at room temperature, 5 mL of methanol and 25 mL of sodium hydrogen carbonate solution were added. The aqueous phase was extracted with DCM (2 x 30 mL), and then the organic fractions were collected, dried over anhydrous sodium sulfate, and the solvent evaporated. The intermediate product was dissolved 33% trimethylamine (TEA) in DCM (50 mL DCM + 25 mL TEA) and stirred overnight at room temperature (to ensure the complete deprotection of cyanoethyl chain). The solvent was evaporated under high vacuum.

The product **1b** was isolated after purification on silica gel (DCM:methanol:TEA from 98:1:1 to 90:9:1).

Yield: 653 mg (37.7 %). *R_{f}* (product **1b**) = 0.3 (DCM:methanoI:TEA 90:9:1).

¹**H NMR** (300 MHz, CDCl₃): *δ* in ppm 0.85 (t, 6H, *J*=6.5 Hz, 2CH₃ of palmitoyl chain), 1.22 (s, 48H, 24CH₂ (palmitoyl chain)), 1.27 (t, 9H, *J*=7.4 Hz, 3CH₃ (triethylammonium)), 1.55 (bs, 4H, 2*CH₂*-CH₂-CO), 2.00-2.34 (m, 5H, 2*CH₂*-CO, H2'(sugar)), 2.51 (bs, 1H, H2"(sugar)), 3.02 (q, *J*=7.2, 3CH₂ (triethylammonium)), 3.25-4.91 (m, 8H, 2CH₂ (glycerol), H3' (sugar), H4'(sugar), 2H5'(sugar)), 5.21 (bs, 1H, -CH- glycerol), 6.02 (d, 1H, *J*=6.5 Hz, H-5(base)), 6.12 (t, 1H, *J*=5.3 Hz, H1'), 8.05 (bs, 1H, H-6 (base)).

**¹³C NMR** (75 MHz, CDCl₃): *δ* in ppm 9.0 (CH₃, triethylammonium), 14.2 (CH₃ chain), 22.8 (*CH₂*-CH₃ chain), 25.0 (*CH₂*-CH₂-C=O), 29.3-29.8 (CH₂ chain), 32.0 (*CH₂*-CH₂-CH₃ chain), 34.2 (*CH₂*-C=O chain), 34.4 (*CH₂*-C=O chain), 45.9 (CH₂ triethylammonium), 61.4 (CH₂, C5'-sugar), 62.8 (*CH₂*-O-C=O glycerol), 63.6 (*CH₂*-O-P=O glycerol), 70.4 (CH glycerol), 74.8 (CH, C3'-sugar), 86.4 (C, C4'-sugar), 95.4 (CH, C5-base), 95.5 (CH, C1'-sugar), 142.1 (CH, C6-base), 155.1 (C=O, C2-base), 162.5 (C, C4-base), 173.2 (C=O chain), 173.6 (C=O chain).

**³¹P NMR** (121 MHz, CDCl₃): *δ* in ppm 1.90. High-resolution ESI MS [M-H]⁻, theoretical m/z=856.54577, observed m/z=856.5459 (0 ppm).

### Exemple 3 : preparation of diC16-3'-dC and diC16-3'-dG liposomes

A method based on thin-film hydration was used.

6 mg of diC16-3'-dC or diC16-3'-dG (prepared in examples 2 and 1) were dissolved and mixed in dichloromethane into 10mL volumetric flask. Aliquots were then prepared by transferring 500µL into a 4mL glass vial. Aliquots were then evaporated until dryness and stored at -20°C.

For diC16-3'-dC, hydration of the lipid film was accomplished by 3 mL water from MΩ water device (ELGA classic) and followed by a heating for 30 min at 60°C. For diC16-3'-dG, the same procedure was followed, except that sonication in an ultrasonic bath (Elmasonic S30H) was performed for 5 min after heating, at the same temperature. The final concentration of diC16-3'-dC or diC16-3'dG in the vial is 100µM (S1).

The particle size was measured by Dynamic Light Scattering (DLS) measurements using a Zetasizer 3000 HAS MALVERN at 25°C with 400 µL of the diC16-3'-dC or diC16-3'-dG solution.

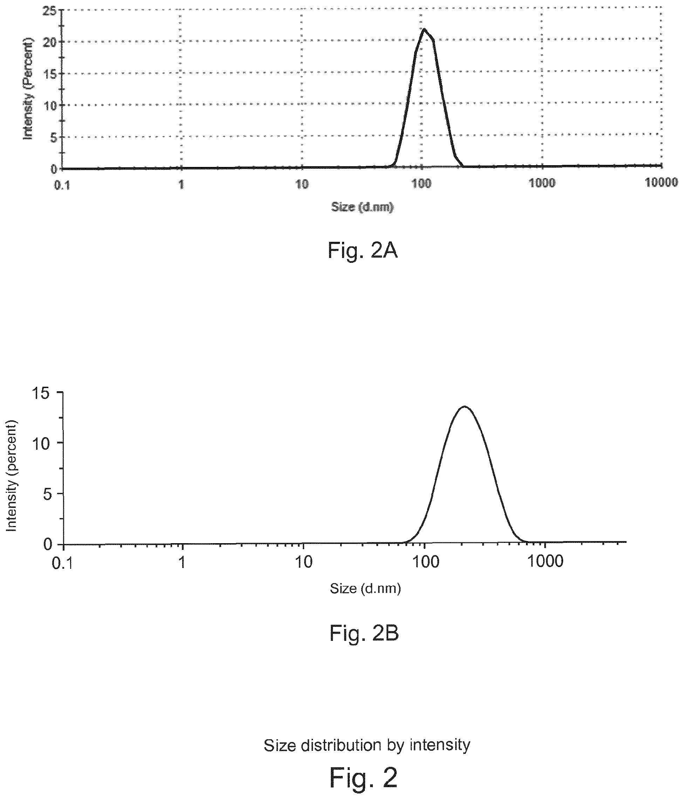

The DLS profiles showing the intensity-averaged diameter are shown on Figure 2.
- diC16-3'-dC (Figure 2A)
Polydispersity index (PdI) = 0.144
Z-Average (d.nm)= = 105.4
- diC16-3'dG (Figure 2B)
Polydispersity index (PdI) = 0.232
Z-Average (d.nm)= = 186.0

### Example 4 :Metal ions detection

The experimental conditions described below apply to the nucleolipids diC16-3'-dC or diC16-3'-dT. For ease of reading, the generic name "diC16-3'-dX" is used below when not referring to a specific nucleolipid.

DiC16-3'-dC was synthesized as described in example 2. DiC16-3'-dT was synthesized according to the litterature procedure (S. Khiati et al., Bioconjugate Chem., 2009, 20, 1765-1772). Dichloromethane, HEPES (4-(2-hydroxyethyl)-1-piperazine ethane sulfonic acid) (Sigma-Aldrich), AgNO₃ (French Pharmaceutical Cooperation), Thioflavin T (Sigma-Aldrich), Dimethyl sulfoxide (DMSO) (PROLABO) and metal salts were used as received without further purification. The metal salts used to assess the specificity are as follows: ZnSO₄, CuSO₄, FeSO₄, NiSO₄, CoSO₄, PbSO₄, HgCl₂, CdSO₄, MnSO₄. MΩ (18.2 MΩ) water from ELGA classic was used for preparing aqueous solutions.

### 1/ Preparation of diC16-3'-dX self-assemblies

The thin-film hydration method described in example 3 for diC16-3'-dC was used for all tested nucleolipids.

### 2/ Preparation of Thioflavine T, HEPES and Metal ions

Thioflavine T (ThT) was prepared by dissolving 4.8 mg of ThT (Sigma-Aldrich) in water (from MΩ water device) into 5 mL volumetric flask (3 mM). This solution was kept in the dark at 4°C. Before the fluorescence assay, a working solution (S2) at 150 µM was prepared from the stock solution (3mM) and kept in the dark.

The HEPES buffer was prepared at 250 mM, pH 7.4 (S3) using 4-(2-Hydroxyethyl)piperazine-1-ethanesulfonic acid (from Sigma-Aldrich) and sodium hydroxide (from Sigma-Aldrich). The pH was measured by a model PHM201 MeterLab pH meter.

The work solutions of metal ions (AgNO₃, HgCl₂) were obtained by series diluting the stock solution (20 mM) in MΩ water to obtain the following concentrations into the wells: 0.1, 0.2, 0.3, 0.4, 0.5, 0.6, 0.7, 0.8, 0.9, 1 µM. In order to obtain these concentrations, solutions at 10, 20, 30, 40, 50, 60, 70, 80, 90, 100µM and 0.2, 0.4, 0.6, 0.8, 1.0, 1.2, 1.4, 1.6, 1.8, 2µM were prepared in prior to use (S4).

### 3/ Preparation of the Metal ions sensing solution

In a 4 mL amber vials, the reagents were introduced in the following order: 2836 µL of Milli-Q water, 24 µL of HEPES from S3, 120 µL of diC16-dX from S1, and finally 20 µL of ThT from S2 with mixing (vortex) after each addition. This way, we obtained a total volume of 3000 µL containing 4 µM of diC16-3'-dX, 1µM of ThT, and 2 mM of HEPES. This solution is used extemporaneously and should be mixed before use (S5).

Then, 100 µL of S5 was transferred into a non-binding microplate well (Grenier bio-one, microplate, 96 well, PS, F-bottom, Chimney well, black, non-binding) of a total volume of 200 µL. Finally, 100 µL of S4 Ag(I) or Hg(II) was added in order to obtain into the wells the following final concentrations: 2 µM of diC16-3'-dX, 0.5 µM of ThT, 1 mM of HEPES and the Ag(I) / Hg(II) concentrations cited above.

During the same day, the fluorescence emission spectra were recorded immediately after an orbital stirring of the microplate of 1 minute, using a TECAN Infinite F500. The bandwidth for emission was 7 nm and the excitation and the emission wavelength were 440 nm and 490 nm, respectively.

### 4/ Results

### 4.1 Measure of ThT fluorescence for a concentration range of Ag+ in presence of diC16-3'-dC liposomes

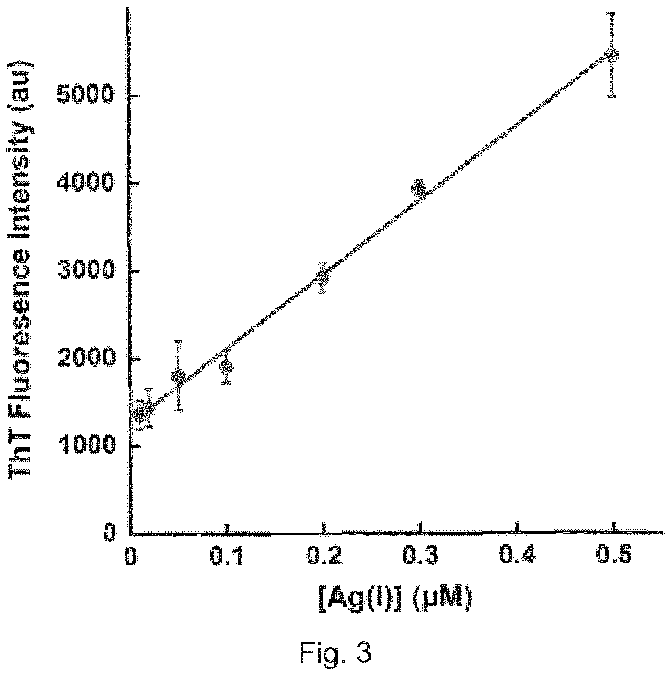

The results are shown on Figure 3.

The ThT fluorescence was measured for a concentration range of Ag+ in presence of diC16-3'-dC liposomes. The line represents the linear regression (R2=0.99) associated with diC16-3'-dC. Error bars are the standard deviation based on triplicate samples.

The results show that a linear fluorescence response (R2=0.99) was observed between 0 and 0.5µM (0 - 50ppb). The limit of detection (LOD) of Ag+ was found to be 50nM (∼5 ppb). It is worth noting the LOD of this simple method is comparable to the LOD of Inductively Coupled Plasma Optical Emission Spectrometry (ICP-OES), and greatly below the regulation threshold of Ag+ in potable water.

### 4.2/ Measure of the THT fluorescence emission with diC16-3'--dC liposomes in the presence of different metal ions

The ThT fluorescence of diC16dC liposomes alone was measured in comparison to the fluorescence in presence of Ag+, or with various metal ions (Ag, Zn, Cu, Fe, Ni, Co, Pb, Hg, Cd, Mn, Hg) at a concentration of 1µM. For each metal ion, experiments were carried out in duplicate.

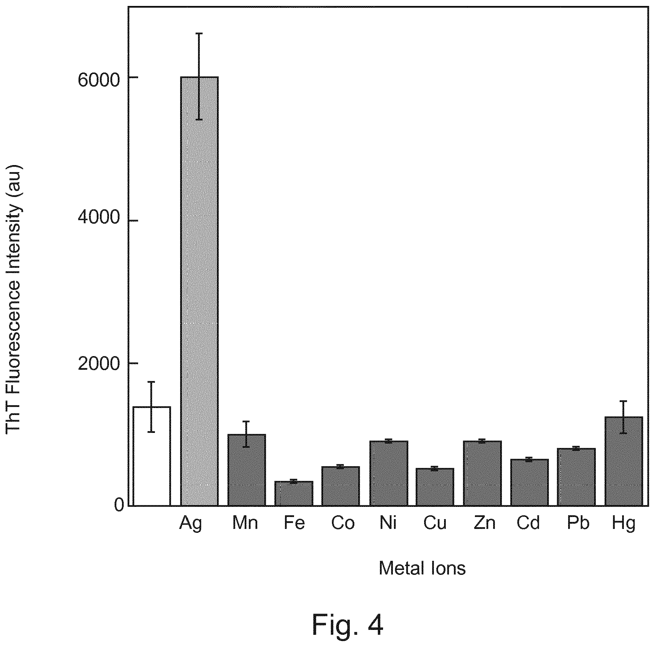

The results are shown on Figure 4.

The results show that the ThT fluorescence, expressed in arbitrary units (au), is selectively increased for the diC16-3'-dC liposomes in the presence of the Ag+ ion. Other metal ions either show no effect (Mn²⁺ and Hg²⁺) or a decrease in ThT fluorescence in the tested conditions.

### 4.3 Measure of the THT fluorescence emission with diC16-3'-dC or diC16-3'-dT liposomes in the presence of Ag⁺ or Hg²⁺

The ThT fluorescence of diC16-3'-dC and diC16-3'-dT liposomes alone (white column) was measured in comparison to the fluorescence in presence of Ag+ (light grey column) or Hg²⁺ (dark grey column). For each metal ion, experiments were achieved in duplicate with the following conditions [HEPES]=1mM pH=7.4; [ThT]=0.5µM; [diC16dX]=2µM; [Ag(I)]=0 or 2µM; [Hg(II)]=0 or 2µM.

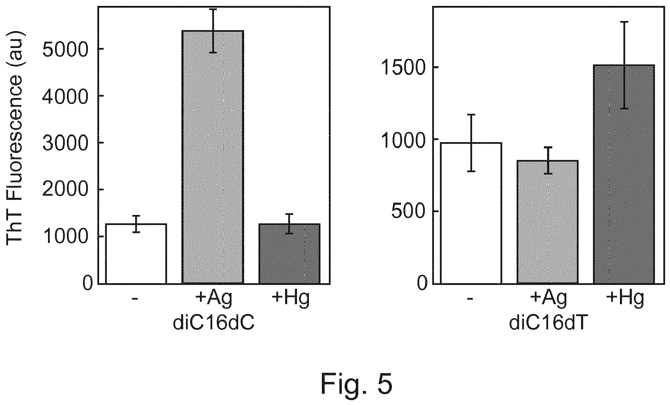

The results are shown on Figure 5.

The results show that diC16-3'-dC shows a specificity with respect to the interaction with Ag⁺ and diC16-3'-dT shows a specificity with respect to the interaction with Hg²⁺.

## Claims

1. A method for metal ion detection in aqueous solutions, comprising:
- contacting an aqueous solution containing at least one metal ion with at least one nucleolipid compound of formula (I) 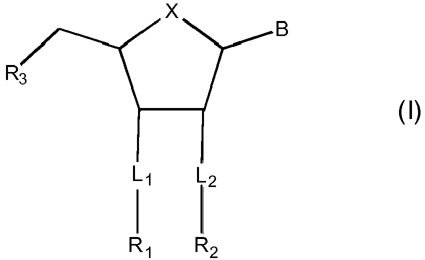 in which
- X is an oxygen atom,
- B is a purine or pyrimidine base, optionally substituted ,
- L₁ is a phosphate group which is substituted by a R₁ group, where R₁ is diacylglycerol in which each acyl group is C₈-C₃₀,
- L₂ is hydrogen,
- R₃ is a hydroxy group,
and
- detecting the presence of the at least one metal ion by measuring the interaction of said at least one metal ion with said at least one nucleolipid compound of formula (I).

2. The method according to claim 1, in which B in formula(I) is cytosine, thymine, guanosine or adenine.

3. The method according to claim 1 or 2, in which R₁ in formula (I) is diacylglycerol in which each acyl group is C₈-C₂₆, preferably C₁₆-C₂₀.

4. The method according to any one of claims 1 to 3, wherein the presence of the at least one metal ion can be detected by using a luminescent probe, in particular a fluorescent probe.

5. The method according to any one of claims 1 to 4, wherein the presence of the at least one metal ion which interacts with nucleolipid compound of formula (I) results in an alteration of the emitted luminescence.

6. The method according to any one of claims 1 to 5, comprising :
- contacting an aqueous solution containing at least one metal ion with a nucleolipid compound of formula (I) as defined in claim 1 and a luminescent probe, and
- measuring the emitted luminescence,
wherein said emitted luminescence results from the interaction of said at least one metal ion with said at least one nucleolipid compound of formula (I).

7. The method according to any one of claims 1 to 5, comprising :
- contacting a sample of an aqueous solution containing at least one metal ion with at least one nucleolipid compound of formula (I) as defined in claim 1 and a luminescent probe,
- measuring the emitted luminescence,
- comparing the emitted fluorescence with that of a control sample of an aqueous solution containing at least one nucleolipid compound of formula (I),
wherein the change of luminescence results from the interaction of said at least one metal ion with said at least one nucleolipid compound of formula (I).

8. The method according to claims 6 or 7, in which the luminescent probe is a fluorescent probe.

9. The method according to any one of claims 1 to 8, in which said interaction consists in the binding of said at least one metal ion to said at least one nucleolipid compound of formula (I).

10. The method according to any one of claims 1 to 9, in which the concentration of said metal ion in the aqueous solution is of 0.05 µM/L to 5 µM/L (5 ppb to 500 ppb).

11. The method according to any one of claims 1 to 10, in which the molar ratio of nucleolipid/metal ion is 0.5 to 10/1, preferably 4/1.

12. The method according to any one of claims 1 to 11, wherein the at least one nucleolipid compound of formula (I) is selected from :
- Thymidine 3'-(1,2-dipalmitoyl-*sn*-glycero-3-phosphate);
- (2*R*, 3*S*, 5*R*)-5-(2-amino-6-oxo-1,6-dihydro-9H-purin-9-yl)-2-(hydroxymethyl)tetrahydrofuran-3-yl ((*R*)-2,3-bis(palmitoyloxy)propyl)-phosphate), and
- (2*R*, 3*S*, 5*R*)-5-(4-amino-2-oxopyrimidin-1(2*H*)-yl)-2-(hydroxymethyl)tetrahydrofuran-3-yl ((*R*)-2,3-bis(tetradecanoyloxy)-propyl)-phosphate.

13. The method according to any one of claims 1 to 12, in which:
- the metal ion is silver (Ag+) and the compound of formula (I) is (2*R*, 3*S*, 5*R*)-5-(4-amino-2-oxopyrimidin-1(2*H*)-yl)-2-(hydroxymethyl)-tetrahydrofuran-3-yl ((*R*)-2,3-bis(tetradecanoyloxy)propyl)-phosphate , or
- the metal ion is mercury (Hg²⁺) and the compound of formula (I) is Thymidine 3'-(1,2-dipalmitoyl-*sn*-glycero-3-phosphate), or
- the metal ion is mercury (Hg²⁺) or copper (Cu²⁺) and the compound of formula (I) is (2*R*, 3*S*, 5*R*)-5-(2-amino-6-oxo-1,6-dihydro-9H-purin-9-yl)-2-(hydroxymethyl)-tetrahydrofuran-3-yl ((*R*)-2,3-bis(palmitoyloxy)propyl)-phosphate.

14. A compound of formula (I) as defined in claim 1, which is selected from :
- (2*R*, 3*S*, 5*R*)-5-(2-amino-6-oxo-1,6-dihydro-9H-purin-9-yl)-2-(hydroxymethyl)tetrahydrofuran-3-yl ((*R*)-2,3-bis(palmitoyloxy)propyl)phosphate;and
- (2*R*, 3*S*, 5*R*)-5-(4-amino-2-oxopyrimidin-1(2*H*)-yl)-2-(hydroxymethyl)tetrahydrofuran-3-yl ((*R*)-2,3-bis(tetradecanoyloxy)propyl)-phosphate.
